(19) Europäisches Patentamt
European Patent Office
Office européen des brevets

(11) Publication number: **0 454 388 A2**

# (12) EUROPEAN PATENT APPLICATION

(21) Application number: **91303572.1**

(22) Date of filing: **22.04.91**

(51) Int. Cl.$^5$: **G01N 33/52,** // G01N33/543

(30) Priority: **23.04.90 IL 94174**

(43) Date of publication of application:
**30.10.91 Bulletin 91/44**

(84) Designated Contracting States:
**DE FR GB**

(71) Applicant: **MAKOR CHEMICAL LTD.**
**Kiryat Weizmann**
**IL-76326 Rehovot (IL)**

(72) Inventor: **Kessler, Igal**
**Ben-Gurion 34/28**
**Rishon Lezion (IL)**

(74) Representative: **W.P. THOMPSON & CO.**
**High Holborn House, 52-54 High Holborn**
**London WC1V 6RY (GB)**

## (54) Apparatus and method for chemical and biological testing.

(57) An apparatus is described for chemical or biological testing for the presence of a material. This comprises an article (20) defining a test surface (16) and at least one chemical or biological agent disposed on the test surface in a spatial arrangement which, when in the presence of the material to be detected, produces a visually readable symbol (e.g. G2b) indicating the test result independent of the spatial arrangement of each chemical or biological agent on the test surface. The symbol for each material to be detected is distinctive relative to symbols used for the detection of other materials. A system for preparing the apparatus may comprise a computer controlled pen plotter (10) and a plurality of replaceable plotting pens (14) associatable with the pen plotter, each of the plotting pens (14) containing a chemical or biological agent to be disposed on a test surface (16).

FIG. 1

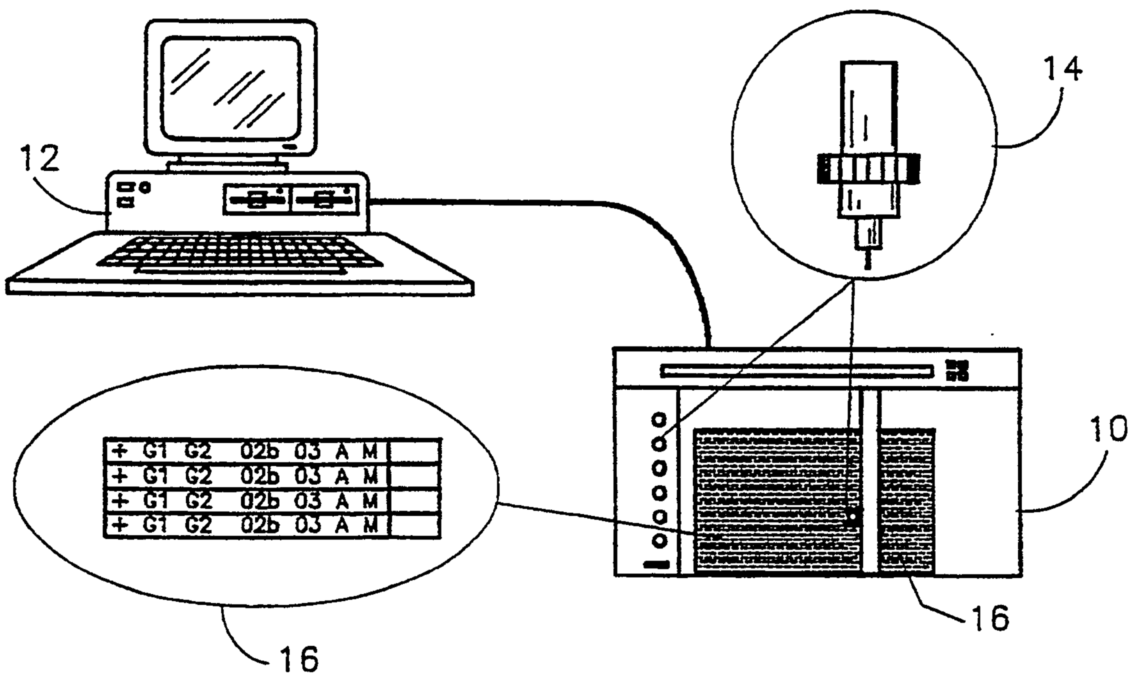


EP 0 454 388 A2

## FIELD OF THE INVENTION

The present invention relates generally to chemical and biological testing.

## BACKGROUND OF THE INVENTION

Various types of apparatus and techniques are known for carrying out chemical and biological testing. In many cases, such as, for example, pH testing, pregnancy testing and immunoassays, specially treated strips are employed for testing and indicate the test results by color changes. In many cases the color changes need to be compared with an external reference in order for the test results to be useful.

There is also known an Amersham Monoclonal Antibody Isotyping Kit, commercially available from Amersham International PLC. of Aylesbury, Bucks. England, wherein a substrate having a plurality of regions, each pre-labeled with an isotype indication is provided. A dot of a different antibody is located in each region. Presence of a given antibody results in a purple indication at the dot in the appropriated labeled region.

Additionally there is available from N.V. Innogenetics S.A. of Ghent Belgium an isotyping kit under the tradename INNO-LIA, which arranges the isotype indications along a strip and enables test result readout without separate readout apparatus.

Another type of isotyping kit is available from SangStat Medical Corporation of Menlo Park, California under the tradename ISO-STAT. This kit employs a test cartridge defining prelabeled regions wherein colored dots appear to indicate the presence of a given isotype.

In an article by Didier Raoult and Gregory A. Dasch entitled: The line blot: an immunoassay for monoclonal and other antibodies, Journal of Immunological Methods 125 (1989) 57 - 65 there is described a procedure for assaying antibodies based on the application of antigen to nitrocellulose as a line with an ink pen point.

## SUMMARY OF THE INVENTION

The Present invention seeks to provide improved apparatus and techniques for chemical and biological testing.

There is thus provided in accordance with a preferred embodiment of the present invention apparatus for chemical or biological testing including an article defining a test surface, at least one chemical or biological agent disposed on the test surface in a pattern which, in the presence of a given material whose presence is to be detected, itself constitutes visually readable information indicating the test result.

In accordance with a preferred embodiment of the present invention the pattern is such that the visually readable information is in alphanumeric form. Alternatively, the pattern is such that the visually readable information is in the form of a universally recognized symbol representing the test result.

According to a preferred embodiment of the invention, the article comprises a generally two dimensional strip and the agent comprises an antibody.

A plurality of agents may be employed, each of which is disposed on the test surface in an arrangement defining different visually readable information. Preferably each agent is written onto the article in a pattern representing the alphanumeric symbol indicating such agent.

Additionally in accordance with a preferred embodiment of the present invention there is provided a method for chemical or biological testing comprising the steps of:

providing an article defining a test surface;

depositing at least one chemical or biological agent on the test surface in an arrangement which, when in the presence of a given material, whose presence is to be detected, produces visually readable information indicating the test result;

exposing the test surface having the agent deposited thereon to a substance to be tested; and

directly reading the test results from the visually readable information produced by an agent in the presence of a given material in the substance tested.

In accordance with a preferred embodiment of the invention, the step of disposing includes disposing at least one agent on the test surface such that the visually readable information is in alphanumeric form.

Additionally in accordance with an embodiment of the invention, the step of disposing includes disposing said at least one agent on the test surface such that said visually readable information is in the form of a universally recognized symbol representing the test result.

Preferably the depositing step includes the step of depositing an antibody on the test surface.

Preferably the depositing step includes the step of depositing a plurality of agents, each of which is disposed on the test surface in an arrangement defining different visually readable information.

Further in accordance with an embodiment of the present invention there is provided a system for preparing

apparatus for chemical or biological testing which comprises an article defining a test surface; at least one chemical or biological agent disposed on the test surface in an arrangement which, when in the presence of a given material, whose presence is to be detected, produces visually readable information indicating the test result, the apparatus for preparing comprising:

a computer controlled pen plotter; and

a plurality of replaceable plotting pens associatable with the pen plotter, each of the plotting pens containing a chemical or biological agent to be disposed on a test surface.

There is also provided in accordance with a preferred embodiment of the invention a method for preparing apparatus for chemical or biological testing which comprises an article defining a test surface; at least one chemical or biological agent disposed on the test surface in an arrangement which, when in the presence of a given material, whose presence is to be detected, produces visually readable information indicating the test result, the method for preparing comprising the step of:

writing with at least one pen containing a chemical or biological agent on said test surface to define an arrangement which produces said visually readable information in the presence of said given material.

Further in accordance with an embodiment of the present invention there is provided for use in a method for preparing apparatus for chemical or biological testing which comprises an article defining a test surface; at least one chemical or biological agent disposed on the test surface in an arrangement which, when in the presence of a given material, whose presence is to be detected, produces visually readable information indicating the test result, apparatus for preparing comprising:

a pen suitable for computer controlled plotting and containing a chemical or biological agent which produces visually readable information in the presence of said given material.

Preferably the chemical or biological agent comprises an antibody.

## BRIEF DESCRIPTION OF THE DRAWINGS

The present invention will be understood and appreciated more fully from the following detailed description, taken in conjunction with the drawings in which:

Fig. 1 is an illustration of a system for preparation of chemical or biological test materials in accordance with a preferred embodiment of the present invention;

Fig. 2 is an illustration of a typical pattern of plotting of antibodies on a test strip;

Fig. 3 is an illustration of a typical test strip prior to development;

Fig. 4 is an illustration of a typical test strip following development;

Figs. 5A, 5B, 5C and 5D illustrate four steps in an indirect enzyme immunoassay (EIA) which may be carried out in accordance with the present invention;

Figs. 6A, 6B, 6C and 6D illustrate four steps in a double antibody (Sandwich) enzyme immunoassay which may be carried out in accordance with the present invention; and

Figs. 7A, 7B and 7C illustrate three steps in a competitive enzyme immunoassay which may be carried out in accordance with the present invention.

## DETAILED DESCRIPTION OF A PREFERRED EMBODIMENT

The present invention seeks to provide apparatus and a technique for biological or chemical testing in which the reaction product appears in the form of a visually readable word or other symbol or collection of symbols which represents the test result.

In accordance with a preferred embodiment of the present invention, a chemically or biologically active agent is written onto a test surface in the form of the word or symbol which is sought to appear as an indication of a given test result.

Preferably, writing of the active agents on test surfaces is carried out using a computer controlled plotter, such as a MP4300 of Graphic Corp. of Tokyo, Japan, which is indicated in Fig. 1 by reference numeral 10. The plotter is controlled by a suitable computer 12, such as an IBM AT. The plotter operation may be governed by any suitable program, typically Autosketch of Autodesk Inc. of the U.S.A. The plotter 10 preferably operates with a plurality of replaceable pens 14, such as Rotring refillable pens, type BC 741310, which provide a 1.0 mm line thickness, each of which is filled with a small quantity of an active chemical or biological agent. Normally each pen 14 contains a different agent.

The technique for production of a mouse monoclonal antibody isotyping test strip in accordance with a preferred embodiment of the present invention will now be described in detail, it being understood that any other suitable test appparatus may also be prepared in accordance with the general technique described hereinbelow, with suitable modifications.

Seven different plotter pen cartridges are each filled, typically with a respective one of the following agents, after dilution of agent A thereof 1:5 which phosphate buffered saline (PBS) which comprises 0.01M phosphate buffer pH 7.4, 0.15M sodium chloride containing 0.1% sodium azide and after reconstitution and dilution 1:10 of agents B - G with PBS. Prior to filling the pens 14, to each 1 ml of each antibody diluted as described above, 0.5 ml of 50% glycerol containing FITC (Flourescein isothiocyanate) was added.

| | | |
|---|---|---|
| 1. | APA to mouse Fab (gt) | BM Product No. X4001. |
| 2. | Anti mouse IgG1 | Sigma Product No. M8144 |
| 3. | Anti mouse IgG2a | Sigma Product No. M8269 |
| 4. | Anti mouse IgG2b | Sigma Product No. M8394 |
| 5. | Anti mouse IgG3 | Sigma Product No. M8519 |
| 6. | Anti mouse IgA | Sigma Product No. M7144 |
| 7. | Anti mouse IgM | Sigma Product No. M7019 |

(BM Products are commercially available from Biomakor of Kiryat Weizmann, Rehovot, Israel; Sigma products are commercially available from Sigma Chemical Co., P.O. Box 14508, St. Louis, Mo. U.S.A.

During operation of the plotter 10 under control from the computer 12, one or more pens 14 write with the active agent or agents on a test surface 16, which during the writing is in the form of a sheet of Mylar, typically of thickness 00 - 100 microns and having formed thereon a nitrocellulose membrane of pore size less than 3 microns. A test surface of this type is commercially available in sheet form from Schleicher & Schuell of D-3354 Dassel, Fed. Republic of Germany.

Using Autosketch software, a plotting area of 3 x 38 strips was designated, each strip being 70 mm long and 7 mm wide. A typical strip is written using active agents in the symbol configuration illustrated in Fig. 2, the numbers underlying each symbol identifying the antibodies listed above, used in writing.

Following plotting, the test surfaces in sheet form are dried, washed with PBS and left in 5% Bovine serum albumin (BSA) containing 0.1% sodium azide, overnight. The next day the sheets are washed with PBS to remove the BSA, dried and cut into strips. The strips are stored under dry conditions at 4 degrees centigrade.

Fig. 3 illustrates a typical test strip 20 ready for use and defining a handle portion 22 and an active portion 24. A typical use protocol employing a double antibody enzime immunoassay to visualize the antibody writing will now be described briefly with reference to Figs. 6A - 6D.

The antibodies plotted on the test strips are each represented as in Fig. 6A. A test strip is placed in a test tube and 2 ml of a tissue culture supernatant test solution, serum or plasma is added to the tube and the tube is stoppered. The tube is incubated horizontally, such that liquid covers the test strip, for 30 minutes. As a result, any recognized antigen attaches to a corresponding antibody, as illustrated in Fig. 6B.

The test strip is then washed and 2 drops of a biotinylated antibody are added to 4 ml PBS and the combination is addded to the tube containing the strip and allowed to incubate for 30 minutes, producing the result shown in Fig. 6C. The test strip is then washed a few times.

Two drops of ExtrAvidin peroxidase reagent (commercially available from Biomakor, Kiryat Weizmann, Rehovot, Israel) are added to 4 ml PBS and added to the tube containing the strip and allowed to incubate for 15 minutes. The test strip is then washed a few times.

Two drops of 1.5M acetate buffer (pH 5.0) are added to 4 ml water and mixed well. One drop of 3-amino-9-ethylcarbazole in D.M.F. is added to the mixture and the mixture is mixed well and thereafter one drip of hydrogen peroxide 2% is added to the mixture and mixed well therewith. The final mixture is added to the tube containing the strip and allowed to incubate, producing the result shown in Fig. 6D.

In the presence of the G2b antibody in the tissue culture supernatant, the strip will appear as illustrated in Fig. 4, bearing a visually readable indication "G2b", approximately 5 -- 10.

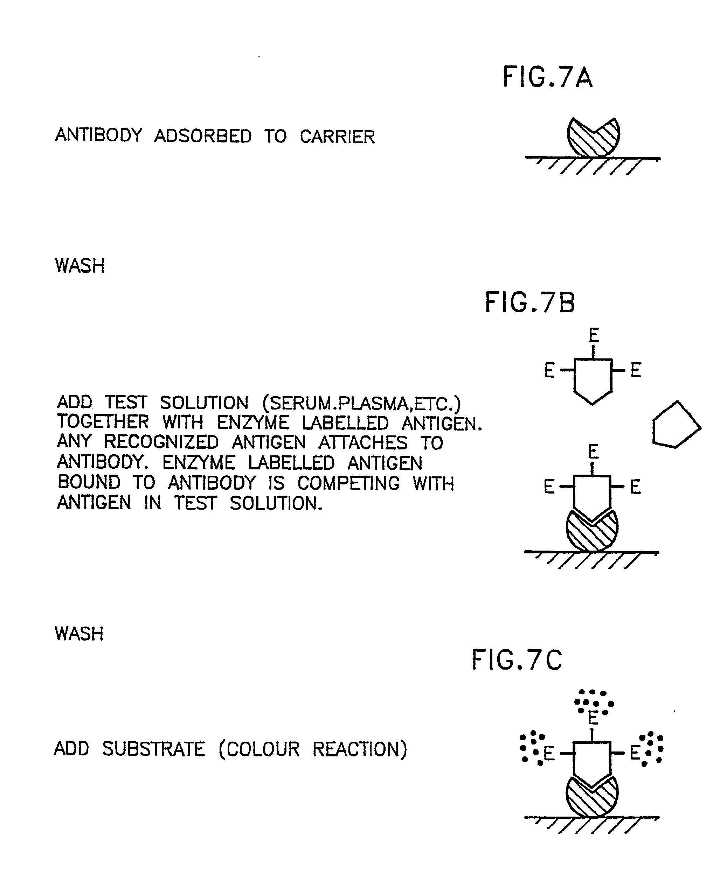

It will be appreciated that other types of immunoassays may alternatively be carried out using the test strip 20. Examples of such other types are illustrated in Figs. 5A - 5D and Figs. 7A - 7C, which also indicate the relevant steps at each stage.

It will be appreciated by persons skilled in the art that the present invention is not limited to immunoassay applications. The invention may be used for any other suitable test. A number of examples of tests employing the invention will now be described.

I. Writing with Antibody (AB)

The following reaction may be provided:

```
AB           AB-AG              AB-AG-AB=E  developing    visual
AB + AG--    AB-AG  +2ndAB=E    AB-AG-AB=E  ------------  indication
AB           AB-AG              AB-AG-AB=E
```

Here the antigen (AG) is the detected substance. The antibody AB is plotted onto the test surface in a visually sensible symbol readout pattern. The second antibody (AB=E) is an enzyme conjugated antibody which enables visualization of the plotted antibody AB.

This type of test can be used, for example to detect a viral infection, in which the antigen AG can be a virus, such as Hepatitis, AIDS, Meningitis, etc. It can be used to detect a microbial infection in which the antigen AG can be any pathogen, such as, for example, Chlamydia, Streptococcus, etc. The text can also be used as a pregnancy test for detecting human chorionic gonadotropin (HCG) in urine. The test may be used generally for detecting any material for which an antibody AB is developed.

II. Writing with Antigen (AG)

The following reaction may be provided:

```
AG           AG-AB             AG-AB-AB*=E  developing    visual
AG + AB--    AG-AB  +AB*=E     AG-AB-AB*=E  ------------  indication
AG           AG-AB             AG-AB-AB*=E
```

Here the antibody (AB) is the detected substance. The antigen AG is plotted onto the test surface in a visually sensible symbol readout pattern. The second antibody (AB*=E) is an enzyme conjugated antibody which enables visualization of the plotted antigen AG.

This type of test can be used, for example to determine the immunological state of humans or animal, for example to determine whether a human is carrying AIDS antibodies or whether a pregnant woman is carrying antibodies to Measles or other viruses.

III. Writing with Enzymes

The following reaction may be provided:

```
E                                E_S               E:P*
E   + S  ------------------------E_S  --------     E:P*
E                                E_S               E:P*
```

Here the detected substance can be a chemical which is also the substrate of one or more enzyme E. The result of the enzymatic activity on the substrate is a visual insoluble product P*.

A mixture of glucose oxidase and peroxidase is plotted onto the test surface in a visually sensible symbol readout pattern. The test surface is then exposed to a blood or urine sample. The glucose oxidase interacts with the glucose in the blood or urine sample to produce hydrogen peroxide which interacts with the peroxidase to create a visual indication on the plotted pattern. This type of test can be semi quantitative and can be used for detection of glucose in diabetics.

IV. Writing with Avidin

The following reaction may be provided:

```
A                                  A=B_X   developing
A    + B_X  ------------------     A=B_X   ---------
A                                  A=B_X
```

This test employs the very high affinity between avidin A, which is plotted on a test surface, and biotin B as the basic recognition event. The test can be used with DNA probes where the DNA probe is biotinylated and is interacted with the plotted avidin. Another possible application is as a genera active surface for interaction with any biotinylated material for applications of the type described hereinabove in examples I, II and III.

It will be appreciated that the invention extends to plotting with any other suitable biologically or chemically active material. For example, the invention may be employed to provide numerical pH indicators in order to obtain a direct numerical visually sensible readout of pH values of a sample on a test strip.

The present invention is not limited by what has been particularly shown and described hereinabove. Rather the scope of the invention is defined by the claims which follow:

## Claims

1. An apparatus for chemical or biological testing for the presence of a material comprising:
   an article defining a test surface, and
   at least one chemical or biological agent disposed on the test surface in a spatial arrangement which, when in the presence of the material to be detected, produces a visually readable symbol indicating the test result independent of the spatial arrangement of each chemical or biological agent on the test surface, the symbol for each material to be detected being distinctive relative to symbols used for the detection of other materials.

2. An apparatus for chemical or biological testing for the presence of a material comprising:
   an article defining a test surface, and
   a plurality of chemical or biological agents disposed on the test surface in a spatial arrangement which, when in the presence of a material to be detected, produce a visually readable symbol indicating the test result independent of the spatial arrangement of each chemical or biological agent on the test surface.

3. An apparatus as set forth in claims 1 or 2 wherein the article is a generally two-dimensional strip.

4. An apparatus as set forth in claims 1, 2 or 3 wherein at least one of the chemical or biological agents is an antibody.

5. An apparatus as set forth in claims 1 or 2 wherein each such visually readable symbol is a numeral, letter, other symbol, or combination thereof.

6. A method for chemical or biological testing for the presence of a material in a substance, the method comprising:
   providing an article having a test surface with at least one chemical or biotogical agent thereon in an arrangement which, when in the presence of the material to be detected, produces a visually readable symbol distinctive for the material to be detected relative to symbols used for the detection of other materials, the visually readable symbol indicating the test result independent of the spatial arrangement of each chemical or biological agent on the test surface,
   exposing the test surface having each such agent deposited thereon to the substance to be tested, and
   directly reading the test results from the visually readable symbol produced on the test surface.

7. A method for chemical or biological testing for the presence of a material in a substance, the method comprising:
   providing an article having a test surface with a plurality of chemical or biological agents thereon in an arrangement which, when in the presence of the material to be detected, produce a visually readable symbol indicating the test result independent of the spatial arrangement of each chemical or biological agent on the test surface,

exposing the test surface having each such agent deposited thereon to the substance to be tested, and

directly reading the test results from the visually readable symbol produced on the test surface.

**8.** A method as set forth in claims 6 or 7 wherein each such visually readable symbol is in alpha, numeric, or alphanumeric form.

**9.** A method as set forth in claims 6, 7 or 8 wherein at least one of the chemical or biological agents is an antibody.

**10.** A system for preparing an apparatus for chemical or biological testing comprising an article defining a test surface having deposited thereon at least one chemical or biological agent in an arrangement which, when in the presence of a material to be detected, produces a visually readable symbol distinctive for the material to be detected relative to symbols used for the detection of other materials, the visually readable symbol indicating the test result independent of the spatial arrangement of each chemical or biological agent on the test surface, the system comprising:

a computer controlled pen plotter, and

a plurality of replaceable plotting pens associatable with the pen plotter, each of the plotting pens comprising a chemical or biological agent to be disposed on a test surface.

**11.** A method for preparing an apparatus for chemical or biological testing comprising:

providing an article having a test surface,

depositing on the test surface at least one chemical or biological agent in an arrangement which, when in the presence of a material to be detected, produces a visually readable symbol distinctive for the material to be detected relative to symbols used for the detection of other materials, the visually readable symbol indicating the test result independent of the spatial arrangement of each chemical or biological agent on the test surface.

**12.** The method of claim 11 wherein said at least on chemical or biological agent is deposited on the test surface by writing with at least one pen containing each such chemical or biological agent.

FIG.1

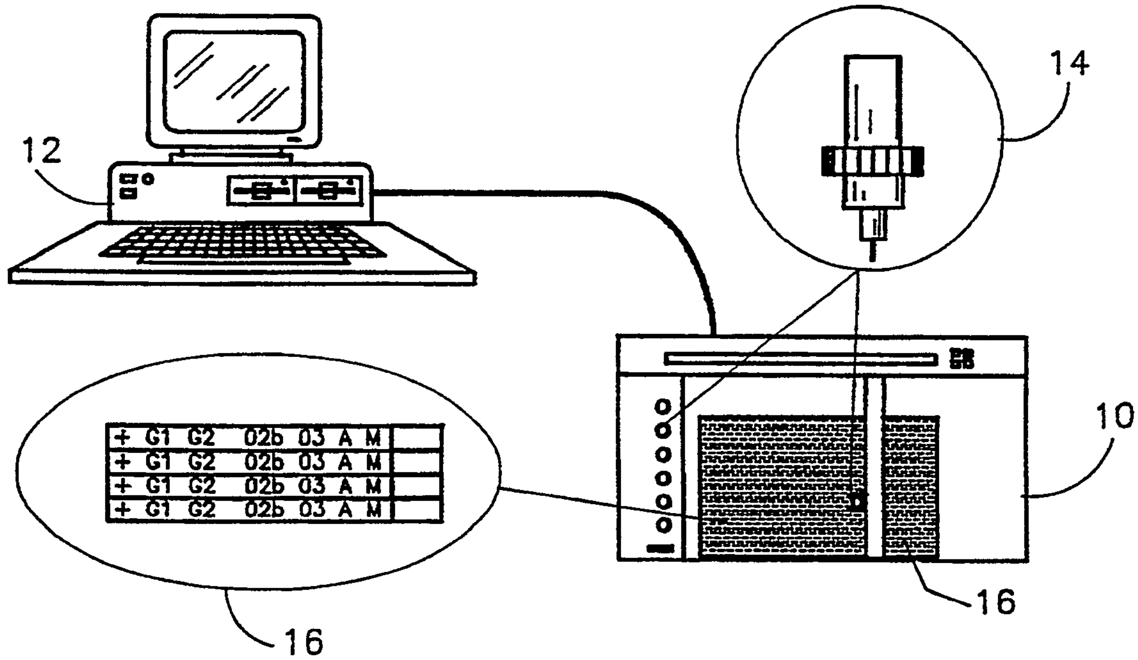


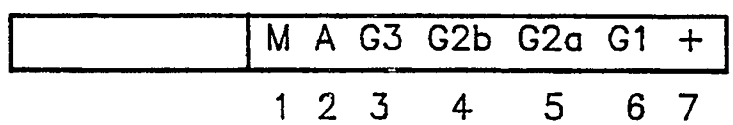


FIG.2

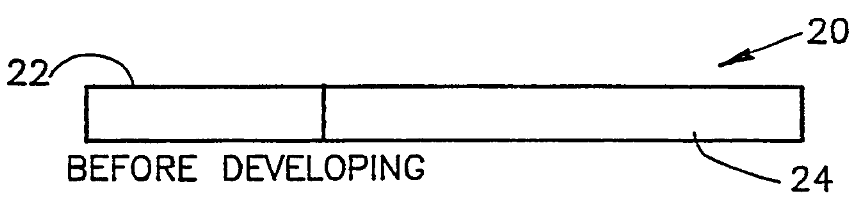


FIG.3

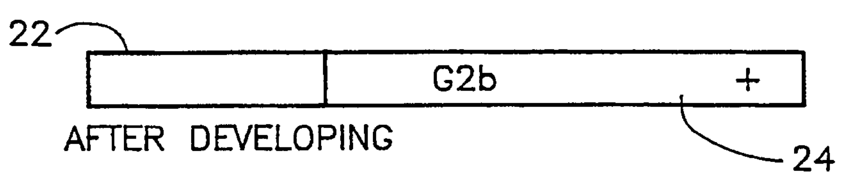


FIG.4

ANTIGEN ADSORBED ON CARRIER

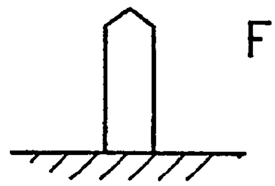

FIG.5A

WASH

ADD TEST SOLUTION (SERUM,PLASMA,ETC.)
ANY SPECIFIC ANTIBODY (1st) ATTACHES
TO ANTIGEN

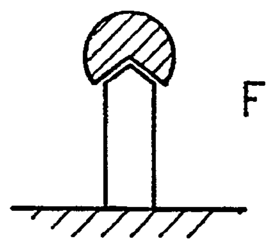

FIG.5B

WASH

ADD ENZYME LABELLED ANTIBODY (2nd)
WHICH ATTACHES TO ANTIBODY (1st)

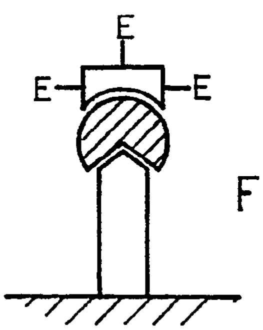


FIG.5C

WASH

ADD SUBSTRATE (COLOUR REACTION)

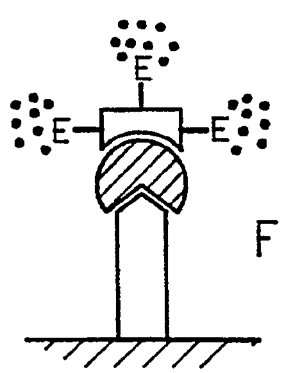


FIG.5D

ANTIBODY (1st) ADSORBED ON CARRIER

FIG.6A

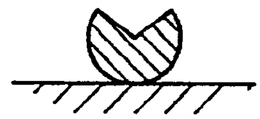

WASH

ADD TEST SOLUTION (SERUM,PLASMA,ETC.)
ANY RECOGNIZED ANTIGEN ATTACHES
TO ANTIBODY (1st)

FIG.6B

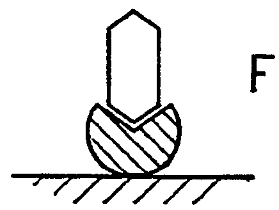

WASH

ADD ENZYME LABELLED ANTIBODY (2nd)
WHICH ATTACHES TO ANTIBODY (AT A
DIFFERENT SITE THEN ANTIBODY (1st))

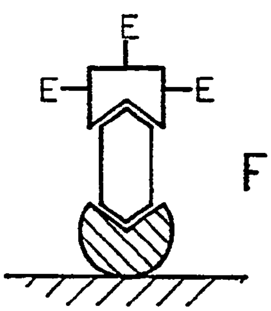


FIG.6C

WASH

ADD SUBSTRATE (COLOUR REACTION)

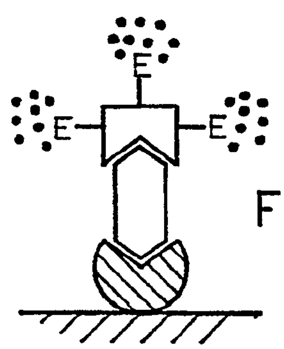


FIG.6D

FIG.7A

ANTIBODY ADSORBED TO CARRIER

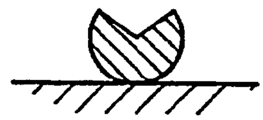

WASH

FIG.7B

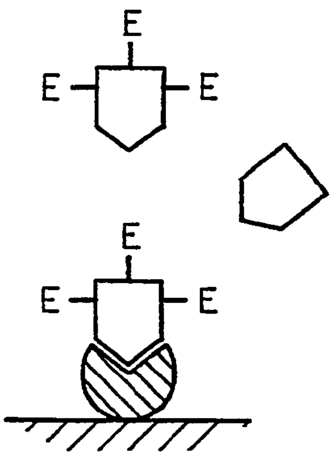


ADD TEST SOLUTION (SERUM.PLASMA,ETC.) TOGETHER WITH ENZYME LABELLED ANTIGEN. ANY RECOGNIZED ANTIGEN ATTACHES TO ANTIBODY. ENZYME LABELLED ANTIGEN BOUND TO ANTIBODY IS COMPETING WITH ANTIGEN IN TEST SOLUTION.

WASH

FIG.7C

ADD SUBSTRATE (COLOUR REACTION)